# EUROPÄISCHE PATENTSCHRIFT

(11) **EP 2 286 780 B1**
(45) Veröffentlichungstag und Bekanntmachung des Hinweises auf die Patenterteilung: **02.04.2014**
(21) Anmeldenummer: 10007128.1
(22) Anmeldetag: 09.07.2010
(51) Int. Cl.: A61F 13/58, A61F 13/60

(54) **Windelverschlussband, Windel sowie Bandmaterial und Wicklung eines derartigen Bandmaterials als Halbzeug**
Nappy closing strip, nappy and strip material and coil of such strip material as semi-finished product
Bande de fermeture de couche, couche ainsi que matériau de bande et enroulement d'un tel matériau de bande comme demi-produit

(30) Priorität: 09.07.2009 DE 102009032666; 11.08.2009 DE 102009036906
(43) Veröffentlichungstag der Anmeldung: 23.02.2011
(73) Patentinhaber: Koester GmbH & Co. KG, 96146 Altendorf (DE)
(72) Erfinder: Neugebauer, Robert, 91077 Neunkirchen (DE); Dittrich, Alfred, 91330 Eggolsheim (DE); Kolic, Jvica, 91336 Heroldsbach (DE)
(74) Vertreter: Reuther, Martin

(56) Entgegenhaltungen:
- EP-A1- 0 941 730
- WO-A1-2006/138016
- US-A- 4 795 456
- US-A- 5 860 964
- US-A1- 2004 170 794
- US-B1- 6 463 633

## Beschreibung

Die Erfindung betrifft ein Windelverschlussband mit einem Befestigungsbereich und einem Nutzerbereich sowie mit einem Übergangsbereich zwischen dem Befestigungsbereich und dem Nutzerbereich, bei welchem der Befestigungsbereich permanent an einer Windel anbringbar ist, indem er wenigstens teilweise an einer Stelle um einen Windelrand gefaltet werden kann. Darüber hinaus betrifft die Erfindung ein Bandmaterial zur Herstellung eines Windelverschlussbandes bzw. ein Bandmaterial mit einem NonWoven als eine Lage zur Herstellung eines Windelverschlussbandes, indem Querstreifen von dem Bandmaterial abgeschnitten werden. Zudem betrifft die Erfindung auch eine Wicklung eines derartigen Bandmaterials und eine entsprechende Windel.

Insbesondere gattungsgemäße Windelverschlussbänder sind aus dem Stand der Technik hinreichend bekannt und werden zum sicheren und einfachen Verschließen von Windeln oftmals mit mechanischen Verschlusseinrichtungen ausgestattet. Auch sind klebende Verschlusseinrichtungen bekannt. Diese Windelverschlussbänder werden beispielsweise an Windelrändern angeklebt oder sonstwie permanent befestigt, welche etwa in Gestalt eines Windelohrs ausgestaltet sein können, wobei die Anbindung beidseitig der Windelränder mit einem Y-Bond oder in einer anderer Weise um den Windelrand herum gefaltet besonders ausreißfest und damit betriebssicher erfolgen kann. Insbesondere wird ein derartiger Windelrand beispielsweise an einem Windelohr zu finden sein. Ebenso kann ein entsprechender Windelrand beispielsweise der äußere Rand eines Backsheets und/oder eines Topsheets bzw. eine angesetzte sonstige Baugruppe, die eine außen liegende Kante bzw. einen außen liegenden Rand aufweist, wie beispielsweise ein entsprechendes Bandteil oder ein Gürtel, sein. Jedoch ergeben sich bei einem diesbezüglichen Umfaltvorgang der Windelverschlussbänder in die Windel hinein immer wieder dahingehend Probleme, dass sich die Windelverschlussbänder bzw. Baugruppen hiervon oftmals nur ungenau umfalten lassen. Insofern kann eine präzise Umfaltposition an den Windelverschlussbändern nicht zuverlässig gewährleistet werden. Dies kann zu Störungen im Herstell- und Verpackungsbetrieb führen, da die Gefahr besteht, dass Teilbereiche der Windelverschlussbänder nach einem Umfaltvorgang immer noch kritisch über die eigentliche Windel hervorstehen.

Hierbei besteht eine Windel in der Regel aus einem Windelkörper, der einerseits - nutzerseitig - saugfähig ausgebildet ist und anderseits - außen - gegen einen Feuchtigkeitsaustritt gesichert ist. Häufig wird dieses durch ein außen angeordnetes Backsheet, welches als dichte Folie ausgebildet ist, die ggf. außen mit einem NonWoven kaschiert ist, sowie ein innen angeordnetes, Feuchtigkeit durchlassendes oder absorbierendes Topsheet umgesetzt, zwischen denen ggf. Feuchtigkeit absorbierendes Material, beispielsweise ein Superadsorber, angeordnet sein kann. Der Windelkörper weist einen äußeren Rand auf, der in der Regel einen bauchseitigen und einen rückenseitigen Bund sowie seitlich, häufig in einem gerafften Bereich, seitliche Beinausschnitte definiert. Von den seitlichen Beinausschnitten ausgehend sind häufig in Richtung auf den Bund Windelohren vorgesehen, an denen die vorstehend erläuterten Windelverschlussbänder angeordnet sind. Diese können beispielsweise an dem Top- bzw. an dem Backsheet, welche an dem Rand miteinander verbunden sind, befestigt werden, derartige Windelohren können jedoch auch einstückig aus dem Windelkörper, insbesondere einstückig aus dem Top- oder Backsheet oder aus beiden, ausgebildet sein. Andererseits können diese - je nach genauer Ausgestaltung der Windel - derartige Windelverschlussbänder auch an anderer Stelle der Windel vorgesehen werden.

Ein gattungsgemäßes Windelverschlussband ist in der Gebrauchsmusterschrift DE 200 23 237 U1 bzw. in dem Europäischen Patent EP 1 255 522 B2 oder in der internationalen Patentanmeldung WO 2001/058402 A1 beschrieben, welches derart an einem Windelrand angeklebt und danach um diesen Windelrand herum gefaltet werden kann, dass es einerseits mit einem ersten distalen Endabschnitt eines Bandlaschenträgers und andererseits mit einem Innenschenkel einer Trennlasche an dem Windelrand befestigt ist, wobei insbesondere der distale Endabschnitt und der Innenschenkel eine besonders ausreißfeste Y-Verbindung bilden. Hierzu wird der Bandlaschenträger derart an dem Windelrand positioniert, dass die Trennlasche unmittelbar vor der Kante des Windelrands platziert ist, wobei der Bandlaschenträger um die Kante des Windelrands herum gefaltet wird und der gefaltete Bereich des Bandlaschenträgers mittels der Trennlasche an der Windelinnenseite klebend gehalten wird. Hierdurch liegt das Windelverschlussband umgefaltet und eingeklappt in die Windel vor, bis ein Benutzer das Windelverschlussband an einem Fingerabziehabschnitt ergreift und ein mechanisches Befestigungsmaterial zur Benutzung aus der Windel heraus klappt.

Ein ebenfalls an einer Windel umfaltbares Windelverschlussband ist aus der internationalen Patentanmeldung WO 2006/138016 A1 bekannt, bei welchem eine Befestigungssektion vor einer Benutzung ebenfalls an einer Windel umgefaltet bzw. um einen Windelrand herum gefaltet angeordnet ist. Auch hier gelingt eine ausreichend präzise Faltung des Windelverschlussbands nur, wenn der umzufaltende Teil des Windelverschlussbandes exakt geführt wird.

In den Druckschriften EP 0 941 730 A1, US 4,795,456, US 6,463,633 B1 und US 6,645,338 B1 ist ein dehnbares Windelverschlussband mit einer nicht dehnbaren Lage beschrieben, wobei die nicht dehnbare Lage zu einem Nutzerbereich, in welchem eine Komponente eines verschließ- und offenbaren Verschlusssystems angebracht ist, führt und eine Separationslinie im Bereich einer dehnbaren Lage aufweist. Die Separationslinie verläuft quer zu der Längserstreckung des dehnbaren Windelverschlussbandes und bewirkt, dass die nicht dehnbare Lage im Bereich der dehnbaren Lage geschwächt und dadurch doch eine gewisse Axialflexibilität erfährt, wenn der Nutzerbereich von einem Nutzer ergriffen und an einer Windel appliziert werden soll, wobei dann der Befestigungsbereich an dem entsprechenden Windelohr und um dieses herumgefaltet verbleibt. Auch dieses dehnbare Windelverschlussband wird mithin in seinem Befestigungsbereich um einen Windelrand herum gefaltet, um so die Windel während der Produktion besser handhaben und insbesondere besser verpacken zu können.

Ein weiteres Windelverschlussband ist in der internationalen Patentanmeldung WO 02/26183 A1 bzw. in der US 6,730,070 B2 beschrieben. Das Windelverschlussband liegt hierbei im Wesentlichen als doppellagige Endlosschleife vor, die mit ihrer Rückseite in eine Innenseite einer Windel eingeklebt wird. An ihrer der Innenseite abgewandten Lage ist eine Perforation vorgesehen, entlang welcher die Lage gezielt getrennt werden kann, so dass ein Windelbefestigungsstreifen mit ersten Befestigungsmitteln herausgeklappt werden kann, wobei die ersten Befestigungsmittel im Gebrauchszustand der Windel mit weiteren Befestigungsmitteln an der Außenseite der Windel korrespondieren können. Durch den hier gewählten Aufbau des Windelverschlussbands wird ein Umfalten des Windelverschlussbands an einem Windelrand bzw. um einen Windelrand herum überflüssig, so dass dementsprechend während der Produktion auch auf einen diesbezüglichen Umfaltvorgang verzichtet werden kann.

Die Windelverschlussbänder werden in der Regel als Haltzeug in er Form von Bandmaterial oder entsprechenden Wicklungen ausgeliefert, wobei dann quer, insbesondere quer zur Maschinenrichtung, Streifen abgeschnitten und an den Windeln in zuvor beschriebener Weise als Windelverschlussbänder appliziert werden können.

Es ist Aufgabe vorliegender Erfindung ein Umfalten eines an einer Windel applizierten Windelverschlussbandes zu verbessern, so dass insbesondere eine exaktere Faltung des Windelverschlussbandes um ein Windelohr vorgenommen werden kann.

Die Aufgabe der Erfindung wird von einem Windelverschlussband mit den Merkmalen des Anspruchs 1 gelöst.

Hierbei kann ein Windelverschlussband mit einem Befestigungsbereich und einem Nutzerbereich sowie mit einem Übergangsbereich zwischen dem Befestigungsbereich und dem Nutzerbereich der Befestigungsbereich permanent an einer Windel anbringbar sein, indem er an einer Stelle um ein Windelrand gefaltet wird, wobei der Übergangsbereich eine Falthilfe in Form einer Materialtrennung aufweist.

Erfindungsgemäß ist das Windelverschlussband mit einer Falthilfe ausgestattet, so dass eine Faltung des Windelverschlussbandes an einer Windel, insbesondere um eine Kante eines Windelrands herum, stets an gleicher Stelle bzw. entlang einer gleichen Faltungslinie und somit mit einer besonders hohen Präzision vorgenommen werden kann, auch wenn möglicherweise eine derartige Präzision an einer Windelmaschine selbst überhaupt nicht vorgesehen ist. Insofern sind die diesbezüglichen Nachteile des Standes der Technik hinsichtlich eines präzisen Umfaltvorgangs abgestellt.

Hierbei kann die Materialtrennung im Voraus, beispielsweise an einer in der Regel wesentlich präziser ausrichtbaren Bandherstellungsmaschine, einfach und betriebssicher bereits vorgesehen sein bzw. werden.

Da Windelverschlussbänder in der Regel als Halbzeug in Gestalt eines Bandmaterials oder entsprechenden Wicklungen ausgeliefert werden, können von einem Bandmaterial zur Herstellung eines Windelverschlussbandes Querstreifen von dem Bandmaterial abgeschnitten werden, wobei das Bandmaterial eine Falthilfe in Form einer längs des Bandes laufenden Materialtrennung aufweist.

Verläuft die Falthilfe längs des Bandmaterials als Materialtrennung können aus dem Bandmaterial besonders effektiv Windelverschlussbänder mit Falthilfen hergestellt bzw. abgeschnitten und das Bandmaterial besonders einfach hergestellt werden.

Andererseits kann auch bei einer Wicklung eines Bandmaterials nach einem der hier geschilderten Merkmale das Bandmaterial in Maschinenrichtung, also in Längserstreckung, gewickelt sein. Die Wicklung kann hierbei in Form einer Rolle oder in Form einer Spule realisiert sein.

Der Begriff "Maschinenrichtung" beschreibt hierbei eine Richtung insbesondere des Bandmaterials, welche im Wesentlichen von der Transportrichtung durch eine Fertigungsmaschine hindurch definiert ist. Während die Maschinenrichtung beim Bandmaterial also im Wesentlichen in Richtung der Längserstreckung des Bandmaterials verläuft, ist die Maschinenrichtung bei einem von dem Bandmaterial abzutrennenden Windelverschlussband im Wesentlichen quer zu der Längserstreckung des Windelverschlussbandes angeordnet.

Als Falthilfe können vorliegend jegliche Materialtrennungen des Windelverschlussbandes in Betracht kommen, die unmittelbar an einem solchen Bandmaterial bzw. Windelschlussband angebracht, vorzugsweise von dem Bandmaterial bzw. Windelverschlussband unmittelbar ausgestaltet, werden können. In diesem Zusammenhang bezeichnet der Begriff "Materialtrennung" jegliche, die Integrität das Materials zerstörende Trennung, wie dieses beispielsweise durch einen Riss, durch ein Entfernen von Material oder durch Schmelzvorgänge bewerkstelligt werden kann.

Eine bevorzugte Ausführungsvariante sowohl hinsichtlich des Windelverschlussbandes als auch des Bandmaterials sieht vor, dass die Materialtrennung ein Schnitt ist.

Beim Windelverschlussband verläuft dieser Schnitt idealerweise im Wesentlichen quer zur Längserstreckung des Windelverschlussbandes. Insofern ist es vorteilhaft, wenn die Falthilfe quer zu einer Längserstreckungsrichtung des Windelverschlussbandes vorgesehen ist.

Besonders hilfsreich kann eine Materialtrennung wirken, wenn die Materialtrennung das Band in seiner Stärke durchtrennt.

Es ist jedoch nicht zwingend, dass das Material zur Gänze in seiner Stärke, also senkrecht zur Maschinenrichtung und Querrichtung, durchtrennt werden muss. Dieses ist aber möglich. Ebenso können aber leichte Rückstände bzw. einzelne Fasern, die nicht oder nicht vollständig durchtrennt wurden, verbleiben, ohne das die Funktion der vorliegenden Falthilfe kritisch beeinträchtigt wird.

Damit insbesondere das Windelverschlussband trotz der Materialtrennung eine noch sehr gute Zugfestigkeit aufweisen kann, ist es vorteilhaft, wenn die Materialtrennung in Maschinenrichtung durch Stege unterbrochen ist, wobei vorzugsweise die Länge der Stege in Maschinenrichtung 0,5% der Länge der Materialtrennung beträgt.

Insbesondere ist es dementsprechend vorteilhaft, wenn die Länge der Stege in Maschinenrichtung 20% der Länge der Materialtrennung nicht übersteigt. Dieses belässt zwar verhältnismäßig wenig Material, welches im Befestigungsbereich bzw. im Übergangsbereich eine Integrität des Windelverschlussbandes gewährleistet. Allerdings kann hierdurch insbesondere sichergestellt werden, kann dass die Faltlinie auch im Bereich der Stege präzise verläuft.

Ist die Materialtrennung durch Stege unterbrochen, so kann bis zur Applikation an der Windel bzw. bis zum Abtrennen des Bandmaterials in einzelne Streifen die Integrität des Bandmaterials erhalten bleiben und dieses leichter verarbeitet werden.

Überraschender Weise hat sich gezeigt, dass trotz einer Durchtrennung oder trotz einer nahezu vollständigen Durchtrennung im Bereich der Falthilfe das Windelverschlussband ausreichend stabil bleibt, wenn es über einen Umfaltvorgang an der Windel befestigt wird. Der Windelrand bzw. das Windelohr sowie ein durch das Windelverschlussband gebildetes Sandwich stabilisieren die Anordnung derart, dass immer noch ausreichend Zugkräfte übertragen werden können.

Besonders bevorzugt sind Materialtrennungen, da diese auch bei hohen bzw. sich ändernden Verarbeitungs- bzw. Herstellungsgeschwindigkeiten reproduzierbare Ergebnisse liefern.

In vorliegendem Zusammenhang bezeichnet der Begriff "Windelrand" jeden Rand einer Windel oder jeder Baugruppe, die an einer Windel angebracht werden soll und die einen entsprechenden Rand aufweist und an welchem ein Windelverschlussband angebracht werden kann. Insbesondere wird ein derartiger Windelrand beispielsweise an einem Windelohr zu finden sein. Ebenso kann ein entsprechender Windelrand beispielsweise der äußere Rand eines Backsheets und/oder eines Topsheets bzw. eine angesetzte sonstige Baugruppe, die eine außen liegende Kante bzw. einen außen liegenden Rand aufweist, wie beispielsweise ein entsprechendes Bandteil oder ein Gürtel, sein.

Das Windelverschlussband weist in der Regel den Befestigungsbereich zur permanenten Befestigung an dem Windelrand und den Nutzerbereich mit einem wieder öffenbaren Befestigungselement auf, wie beispielsweise eine Komponente eines zweikomponentigen Haken-Schlaufenverschlusses oder wie beispielsweise einen nicht permanent haftenden Klebstoff. Zwischen dem Befestigungsbereich, also dem Bereich, der permanent an der Windel bzw. an dem Windelrand befestigt ist, und dem Nutzerbereich mit einem wieder öffenbaren Befestigungselement kann ein Zwischenbereich vorgesehen sein, der letztlich eine freie Beweglichkeit des Nutzerbereichs und/oder über einen elastischen Bereich eine Elastizität des Windelverschlussbandes gewährleistet. In vorliegendem Zusammenhang wird ein derartiger Zwischenbereich als zum Nutzerbereich gehörig definiert. Eine klare Trennungslinie zwischen dem Befestigungsbereich und dem Nutzbereich kann in der Regel nicht gezogen werden, dieses ist schon naturgemäß durch Toleranzen während des Herstellungsprozesses bedingt. Insofern ist zwischen dem Befestigungsbereich und dem Nutzerbereich ein Übergangsbereich vorgesehen, der möglicherweise nicht eindeutig dem Befestigungsbereich und/oder dem Nutzerbereich zugeordnet werden kann. Dieser Übergangsbereich kann beispielsweise Bereiche des Nutzerbereichs, insbesondere eines von dem Nutzbereich umfassten Zwischenbereichs, umfassen, die ebenfalls permanent an der Windel befestigt sind jedoch nicht klar dem Befestigungsbereich zugeordnet werden können. Auch kann der Übergangsbereich Bereiche des Befestigungsbereichs umfassen, die nicht permanent an der Windel befestigt sind, aber nicht eindeutig dem Nutzerbereich zugeordnet werden können. Angesichts der üblichen Toleranzen bei der Fertigung derartiger Windelverschlussbänder wird der Übergangsbereich in der regel jedoch 20% der Breite des Befestigungsbereichs bzw. 4mm absolute Breite nicht übersteigen.

Die Erfindung geht hierbei auch von der Grunderkenntnis aus, dass eine Sollbruchstelle, die bei Windelverschlussbändern als nicht wieder verschließbare Verbindung bereits bekannt ist, als Falthilfe genutzt werden kann, ohne die Fähigkeit des Windelverschlussbandes und seiner Befestigung an dem Windelrand, Zugkräfte zu übertragen, maßgeblich zu beeinträchtigen, da durch das Umfalten eine entsprechende Stabilisierung erfolgt.

Des Weiteren ist es außergewöhnlich vorteilhaft, wenn die Materialtrennung ein NonWoven durchtrennt, welches eine Lage des Windelverschlussbandes bildet. Beispielsweise ist das NonWoven eine Trägerbahn des Windelverschlussbandes. Hierbei kann die Materialtrennung auf besonders einfache und betriebssichere Weise insbesondere bei einem NonWoven, welches durch seine Fasern nur sehr schwer präzise faltbar ist, eine exakte Faltlinie gewährleisten. Andererseits kann - je nach konkreter Umsetzung vorliegender Erfindung - eine Materialtrennung auch bei anderen Materialien, wie insbesondere bei Folien, beispielsweise aus Kunststoff, oder bei Papier als Falthilfe genutzt werden.

NonWoven werden - anders als Papier - allenfalls sehr locker flächig verpresst, während Papier - schon um eine gute Bindung der einzelnen Zellulosefasern zu erzielen - einer hohen Flächenpressung unterzogen wird. Diese sorgt für einen im Wesentlichen gleichförmigen Verbund, während Fasern bei einem NonWoven teilweise gar nicht miteinander verbunden sind, so dass lediglich Oberflächeneffekte, wie etwa van-der-Vaals-Kräfte, für eine innige Verbindung sorgen, wobei ggf. durch die Verbindung mit weiteren Lagen dann eine Stabilisierung der NonWovenlage dann gewährleistet werden kann. Bei anderen NonWoven können punktuelle Verschweißungen vorliegen, welche jedoch abseits dieser punktuellen Verschweißungen gerade sehr wenig miteinander verbundene Fasern umfassen, damit der flauschige Charakter des NonWoven erhalten bleibt. Selbiges kann beispielsweise durch ein mittels Wasserstrahl verfestigtes NonWoven sichergestellt werden, bei welchem die Fasern mittels eines Wasserstrahls, welcher die NonWovenlage durchdringt, ähnlich einem Filzvorgang miteinander verbunden werden. Insofern fehlt bei einem NonWoven oftmals bzw. in Regel eine Verdichtung, wie sie aus dem Papierbereich bekannt ist.

Da insbesondere bei einem NonWoven schon eine Materialschwächung als Falthilfe ausreichen kann, kann bei einem Windelverschlussband mit einem NonWoven als einer Lage die Falthilfe in Form einer Materialschwächung vorgesehen sein.

In diesem Zusammenhang bezeichnet der Begriff "Materialschwächung" jedgliche lokale Verminderung der Eigensteifigkeit des jeweiligen Materials, wie dieses beispielsweise durch eine Rillung oder eine punktuelle starke Kompression realisiert werden kann. Ebenso können jedoch auch Schweißungen oder Materialtrennungen entsprechend wirksam sein.

Zum anderen kann bei einem Bandmaterial mit einem NonWoven als eine Lage zur Herstellung eines Windelverschlussbandes sich das Bandmaterial durch eine Falthilfe in Form einer längs des Bandmaterials laufenden Materialschwächung auszeichnen.

Auch kann in diesem Zusammenhang bei einem Windelverschlussband mit einem Befestigungsbereich und mit einem Nutzerbereich sowie mit einem Übergangsbereich zwischen dem Befestigungsbereich und dem Nutzerbereich der Befestigungsbereich permanent an einer Windel anbringbar sein, indem er an einer Stelle um einen Windelrand gefaltet wird, wobei der Übergangsbereich eine Falthilfe in Form einer Materialschwächung von mindestens 50% aufweist.

Bei einer Materialschwächung von mindestens 50% kann bei NonWoven als zu faltenden Material bereits schon eine deutliche Tendenz festgestellt werden, sich entlang der Materialschwächung zu falten, so dass eine entsprechende Materialschwächung als Falthilfe genutzt werden kann.

Hinsichtlich eines Halbzeugs für Windelverschlussbänder können bei einem Bandmaterial zur Herstellung eines Windelverschlussbandes Querstreifen von dem Bandmaterial abgeschnitten werden, wobei das Bandmaterial eine Falthilfe in Form einer längs des Bandmaterials laufenden Materialschwächung von mindestens 50% aufweist.

Die Materialschwächung kann beispielsweise durch eine Materialkomprimierung, wie beispielsweise eine Rillung, umgesetzt sein bzw. eine Materialverdrängung, wie beispielsweise einen Falz, umfassen. Als Materialschwächung können aber auch gravierendere Prozesse, wie beispielsweise entsprechende Schweißlinien oder die vorstehend bereits im Detail erläuterten Materialtrennungen bzw. Schnitte oder Einschnitte, genutzt werden.

Die Falthilfe kann dementsprechend vorteilhaft weiterentwickelt werden, wenn die Materialschwächung eine Materialtrennung ist, insbesondere wenn die Materialtrennung ein Schnitt ist und/oder durch Stege unterbrochen ist, wie vorstehend bereits beschrieben ist. Auch hierbei muss die Materialtrennung das NonWoven nicht zwingend zur Gänze durchtrennen.

Auch die Materialschwächung kann durch Stege unterbrochen sein, wobei die Länge der Stege in Maschinenrichtung wenigstens 0,5% der Länge der Materialschwächung und vorzugsweise nicht über 20% der Länge der Materialschwächung betragen kann. Trotz der Stege kann auch bei einen NonWoven dann eine ausreichende Faltgenauigkeit erzielt werden.

Je nach konkreter Umsetzung vorliegender Erfindung können sowohl Materialschwächung als auch Materialtrennung als Falthilfe vorliegen. So kann eine in der Stärke des Bandmaterials unvollständige Materialtrennung zu einer Materialschwächung, insbesondere beispielsweise zu einer Verdichtung oder Komprimierung des restlichen Materials führen, was eine Faltung zusätzlich präzisieren kann. Auch können im Bereich verbleibender Stege zwischen Materialtrennungen entsprechend Materialkomprimierungen als Materialschwächungen vorgesehen sein, um auch im Bereich der Stege ein Falten erfindungsgemäß auszurichten.

Durch die vorliegende Materialtrennung oder eine entsprechend ausgestaltete Materialschwächung, die beispielsweise auch als Schnitt, als Perforation oder ähnliches ausgestaltet sein kann, erfolgt bei der Herstellung des Windelverschlussbandes bzw. des entsprechenden Halbzeugs die Bereitstellung einer Falthilfe, wodurch vorteilhafter Weise eine höhere Faltgenauigkeit erzielt werden kann, als ein Falten an der Windelmaschine selbst ohne eine in dem Material vorgesehene Falthilfe. Die Falthilfe führt vorteilhafter Weise zu einer wesentlich genaueren Faltung an der Windelmaschine.

Besonders vorteilhaft ist es in diesem Zusammenhang, wenn an einem Bandmetrial, das als Halbzeug zum Abtrennen von Windelverschlussbändern genutzt werden soll, eine Materialschwächung und/oder eine Materialtrennung in Gestalt einer Falthilfe realisiert ist, welche kürzer ausgebildet ist als eine Breite eines abzutrennenden Querstreifens bzw. Windelverschlussbandes. Auf diese Weise wird eine Umfalten an entsprechender Stelle erleichtert, ohne dass der Querstreifen bzw. das Windelverschlussband völlig durchtrennt ist, so dass nachwievor eine einfache Materialführung gewährleistet werden kann. Variationen der Länge der Materialschwächung und/oder Materialtrennung können hierbei sehr groß sein und die parallel zur Maschinenrichtung des Bandmaterials verlaufende Breite des Windelverschlussbandes unter Umständen weit übersteigen, solange die Integrität des Bandmaterials gewährleistet werden kann, da es, je nach konkreter Wickelmaschine, denkbar ist, das Windelverschlussband in der kurzen Zeit zwischen der Abtrennung von dem Bandmaterial und der Applikation an der Windel durch Maßnahmen an der Windelmaschine geeignet zu fixieren bzw. zu positionieren.

Ist die Falthilfe befestigungsbereichseitig mehr als 0,3 mm, vorzugsweise mehr als 0,5 mm, von einem Klebebereich eines Releasetapes entfernt angeordnet, kann eine Faltung um eine Kante einer Windelecke bzw. eines Windelrands herum besonders vorteilhaft vorgenommen werden.

In diesem Zusammenhang ist es vorteilhaft, wenn die Falthilfe befestigungsbereichseitig weniger als 2 mm, vorzugsweise weniger als 1 mm, von einem Klebebereich eines Releasetapes entfernt angeordnet ist, damit das Windelverschlussband trotz Falthilfe immer noch ausreichend kompakt ausgelegt werden kann.

Die Vorzüge vorliegender Erfindung können besonders geeignet genutzt werden, wenn der Übergangsbereich ebenfalls wie der Befestigungsbereich des Windelverschlussbandes zumindest im Bereich der Falthilfe ebenfalls permanent an der Windel bzw. an dem Windelrand befestigt ist.

Auch eine Windel mit einem vorstehend beschriebenen Windelverschlussband nach einem der hier beschriebenen Merkmale kann mit einer wesentlich höheren Qualität bereit gestellt werden.

Es versteht sich, dass das Windelverschlussband in vielfältiger Weise an die Windel appliziert und befestigt werden kann. In der Praxis hat es sich bewährt, wenn das Windelverschlussband mittels eines Y-Bonds an der Windel befestigt ist. Auch Variationen hiervon, beispielsweise mittels eines Verklebungs- oder Kaschierbandes, sind möglich. Hierdurch kann das Windelverschlussband einen Windelrand, wie beispielsweise ein Windelohr, im Wesentlichen in Gestalt einer Sandwichanordnung umschließen. Insofern ist das Windelverschlussband besonders ausreißfest an einem Windelrand der Windel befestigt.

Vorteilhafter Weise liegt die Falthilfe nach der Applikation an der Windel an einem Windelrand der Windel an, so dass trotz der Materialtrennung bzw. trotz der Materialschwächung eine ausreichend stabile Verbindung zwischen Windel und Windelverschlussband im Befestigungsbereich bzw. im Übergangsbereich zu gewöhnlichen Betriebssituationen gewährleistet werden kann.

Darüber hinaus ist es vorteilhaft, wenn das Windelverschlussband mittels einer Permanentverbindung, wie beispielsweise mittels einer Klebstoffschicht, einer Ultraschallschweißverbindung o.ä., an der Windel bzw. an einem Windelrand der Windel befestigt ist und in Längserstreckungsrichtung des Windelverschlussbandes gesehen beidseits der Falthilfe die Permanentverbindung vorgesehen ist. Auf diese Weise kann kumulativ bzw. alternativ zu dem vorgenannten Merkmal eine ausreichend stabile Befestigung zwischen Windel und Windelverschlussband im Befestigungsbereich bzw. im Übergangsbereich gewährleistet werden.

Weitere Ziele, Vorteile und Eigenschaften vorliegender Erfindung werden anhand nachfolgender Beschreibung anliegender Zeichnung erläutert, in welcher beispielhaft ein Windelverschlussband, ein diesbezügliches Bandmaterial und eine diesbezügliche Wicklung dargestellt sind. Es zeigen:
- Figur 1: schematisch ein geöffnetes Windelverschlussband mit einer Falthilfe, welches an einem Windelohr appliziert ist;
- Figur 2: schematisch eine Seitenansicht des Windelverschlussbandes aus der Figur 1, welches noch nicht an dem Windelohr appliziert ist;
- Figur 3: schematisch eine Seitenansicht des Windelverschlussbandes aus den Figuren 1 und 2, welches in eine geschlossenen Position an dem Windelohr umgefaltet ist;
- Figur 4: schematisch eine Aufsicht auf einen Teilabschnitt eines Bandmaterials mit einer Falthilfe;
- Figur 5: schematisch eine Ansicht einer Wicklung des Bandmaterials aus der Figur 4; und
- Figur 6: schematisch eine Seitenansicht eines weiteren Windelverschlussbandes, welches noch nicht an dem Windelohr appliziert ist, in ähnlicher Darstellung wie Figur 2.

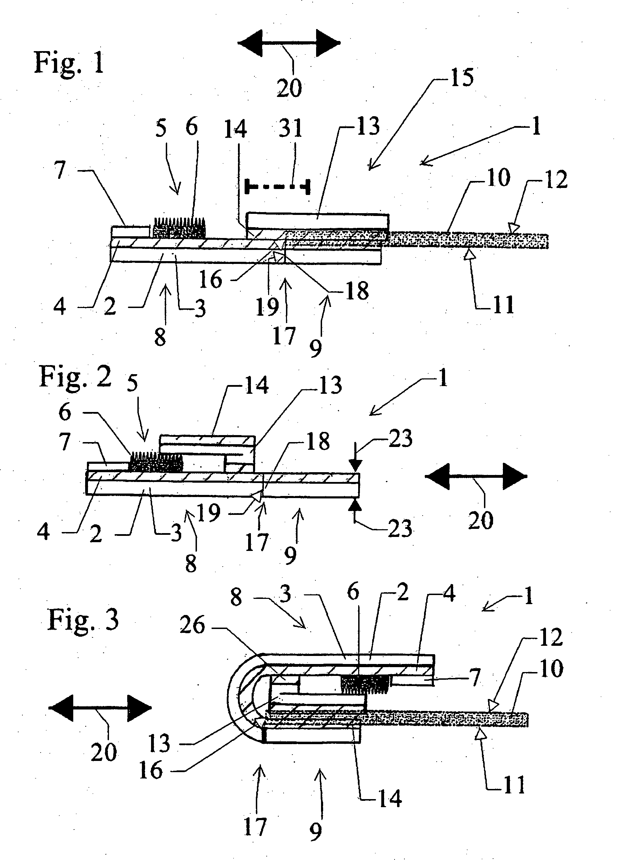

Das in der Figur 1 gezeigte Windelverschlussband 1 weist einen einfachen Aufbau mit einer Trägerbahn 2 auf, die als NonWoven 3 ausgestaltet ist. Die Trägerbahn 2 ist mit einem Klebstoff 4 ausgestattet, mittels welchem eine mechanische Befestigungseinrichtung 5 in Gestalt eines Hakenmaterials 6 und ein Fingerlift 7 auf die Trägerbahn 2 aufgeklebt sind. Hierbei versteht es sich, dass in abweichenden Ausführungsformen alternativ bzw. kumulativ zu dem Hakenmaterial auch unmittelbar Klebstoff oder Bestandteile eines anderen Verschlusssystems entsprechend zur Anwendung kommen können. Ebenso ist es denkbar, dass auf den Fingerlift verzichtet oder dieser auf andere Weise ausgebildet wird.

Insbesondere mittels der mechanischen Befestigungseinrichtung 5 und des Fingerlifts 7 ist ein Nutzerbereich 8 des Windelverschlussbandes 1 festgelegt, welchem ein Befestigungsbereich 9 des Windelverschlussbandes 1 gegenüberliegt.

Während der Nutzerbereich 8 eine Verschlussseite des Windelverschlussbandes 1 definiert, ist das Windelverschlussband 1 mit seinem Befestigungsbereich 9 dauerhaft an einem Windelohr 10 einer hier nicht näher dargestellten Windel angeklebt. Es versteht sich, dass statt eines Windelohrs 10 auch ein anderer Windelrand, wie beispielsweise ein separat angesetztes Windelohr, der äußere Rand eines Backsheets und/oder eines Topsheets bzw. eine angesetzte sonstige Baugruppe, die eine außen liegende Kante bzw. einen außen liegenden Rand aufweist, entsprechend genutzt werden kann.

Hierbei ist die Trägerbahn 2 mittels des Klebers 4 an einer Außenseite 11 des Windelohrs 10 angeklebt. Um zwischen dem Windelverschlussband 1 und dem Windelohr 10 eine hohe Ausreißsicherheit sowie eine betriebssichere Lagerung des Nutzerbereichs 8 an dem Windelohr 10 in einer Bereitstellungsposition gewährleisten zu können, ist an einer Innenseite 12 des Windelohrs 10 ein Releasetape 13 mittels einer Kleberschicht 14 aufgeklebt. Insofern ist das Windelohr 10 von dem Windelverschlussband 1 in Art eines Sandwiches zwischen der Trägerbahn 2 und dem Releasetape 13 klebend eingeschlossen, so dass sich der Befestigungsbereich 9 durch einen Y-Bond 15 auszeichnet, der die hohe Ausreißsicherheit des Windelverschlussbandes 1 gewährleistet.

Um das Windelverschlussband 1 um den Endbereich 16 des Windelohrs 10 besonders gut herumfalten zu können, weist das Windelverschlussband 1 erfindungsgemäß eine Falthilfe 17 zum kontrollierten Falten des Windelverschlussbands 1 um den Endbereich 16 herum auf. Die Falthilfe 17 ist im Wesentlichen befestigungsbereichseitig an dem Windelverschlussband 1 vorgesehen und durch eine Materialschwächung 18 in Gestalt einer Materialtrennung 19 insbesondere durch das NonWoven 3 umgesetzt.

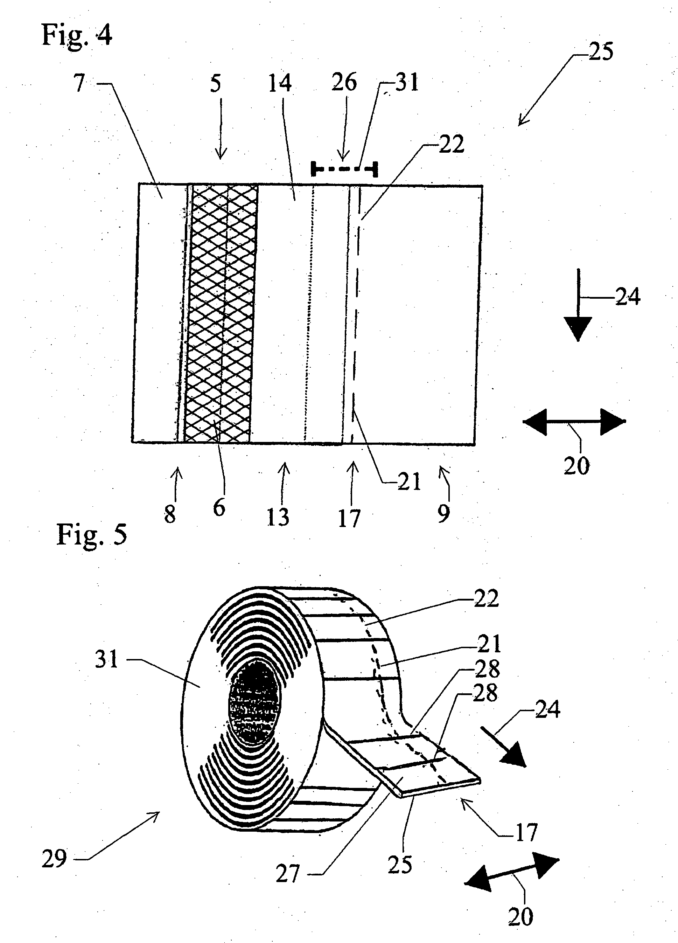

Die Materialtrennung 19 liegt hierbei quer zur Längserstreckung 20 des Windelverschlussbandes 1 als Schnitte 21 (siehe insbesondere Figur 4) vor und beträgt, wie auch die Materialschwächung 18, hierbei offensichtlich mindestens 50% der Materialstärke, wobei es sich versteht, dass naturgemäß bei der industriellen Fertigung einige ungeschnittene Fasern des NonWoven verbleiben können und dass das NonWoven während des Schneidens, insbesondere auch im Bereich von Stegen 22, komprimiert als hinsichtlich seiner Stärke geschwächt, sein kann.

Mittels der Schnitte 21 ist das Windelverschlussband 1 bis auf Stege 22 (siehe insbesondere Figur 4) in seiner Stärke 23 (siehe Figur 2) durchtrennt. Oftmals genügt es, wenn lediglich eine einzelne Lage, wie etwa die Trägerbahn 2, des Windelverschlussbandes 1 eine derartige Falthilfe 17 umfasst.

Die Stege 22 überbrücken hierbei senkrecht zur Maschinenrichtung 24 (siehe Figuren 4 und 5), welche wiederum quer zur Längserstreckung 20 des Windelverschlussbandes 1 definiert ist, die Materialtrennung 19. Die Länge der Stege 22 in Maschinenrichtung 24 beträgt wenigstens 0,5% der Länge der Materialtrennung 19 in Maschinenrichtung 24 gesehen.

In der Figur 2 ist das Windelverschlussband 1 in einer noch unapplizierten Position dargestellt, wobei es bereits von einem Bandmaterial 25 (siehe Figur 4) abgeschnitten vorliegt. Die Falthilfe 17 ist vorliegend 1 mm von einem Klebebereich 26 des Releasetapes 13 entfernt angeordnet.

In der Figur 3 ist das Windelverschlussband 1 an das Windelohr 10 appliziert und um diese herum umgefaltet, wobei die Falthilfe 17 an dem Windelohr 10 anliegt. Mittels der Falthilfe 17 kann ein Umfalten des Windelverschlussbandes 1 um einen Endbereich 16 des Windelohrs 10 wesentlich leichter und präziser vorgenommen werden. Insofern kann hierdurch ein außergewöhnlich betriebssicherer Produktionsablauf und eine besonders hohe Produktqualität sichergestellt werden.

Um die Windelverschlussbänder 1 aus dem Bandmaterial 25, wie es gemäß der Darstellung nach der Figur 4 gezeigt ist, gewinnen zu können, werden von dem Bandmaterial 25 entsprechende Querstreifen 27 - in der Figur 5 mittels Schnittlinien 28 angedeutet - abgeschnitten, wobei das Bandmaterial 25 hierbei kontinuierlich von einer Rolle 29 abgespult wird.

Das Bandmaterial 25 ist hierbei als Halbzeug in Gestalt einer Wicklung 30 bereits mit der Materialtrennung 19 aus Schnitten 21 und Stegen 22 ausgestattet und kann so direkt vor dem Applizieren an dem Windelohr 10 der Windel in die entsprechenden Windelverschlussbänder 1 geschnitten werden.

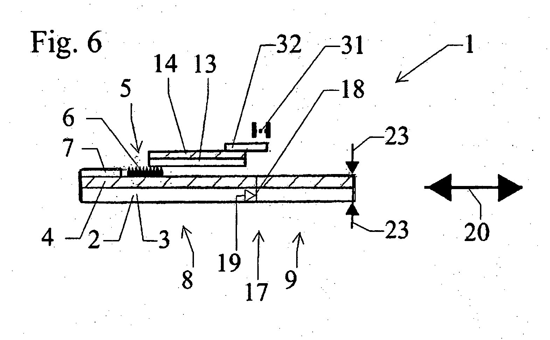

Das in Figur 6 dargestellte Windelverschlussband 1 entspricht im Wesentlichen dem zuvor dargestellten und erläuterten Windelverschlussband 1, so dass gleiche bzw. gleich wirksame Baugruppen auch gleich beziffert sind. Aus diesem Grunde wird auch auf eine erneute Detailerläuterung verzichtet, um Wiederholungen zu vermeiden.

Während jedoch das insbesondere in Figur 2 dargestellte Windelverschlussband 1, welches in dieser Form auch in Rollenform gelagert und verschifft werden kann, ein umgefaltetes Releasetape 13 aufweist, welches letztlich erst bei Verschließen der Windel verstreckt wird (siehe Figur 1), kann das Releasetape 13 des Windelverschlussbands 1 nach Figur 6 in gestrecktem Zustand in Form einer Rolle gelagert und verschifft werden. Bei der Applikation an einer Windel bzw. an einem Windelrand, erfolgt eine Faltung des Windelverschlussbandes 1 an der Falthilfe 17 um den Endbereich 16 (siehe Figur 1) herum.

Hierbei kann in einer ersten Umsetzung dieses Ausführungsbeispiels das Releasetape 13 ungefaltet auf der Innenseite 12 zur Anlage kommen. Bei eine Strecken des Windelverschlussbandes, beispielsweise wenn die Windel verschlossen werden soll, wie dieses beispielhaft in Figur 1 angedeutet ist, verbleibt das Releasetape 13 dann zur Gänze an der Innenseite 12. Die Integrität des Windelverschlussbandes 1 wird bei dieser Umsetzung im Bereich der Falthilfe 17 zur Gänze durch die Trägerbahn 2 sowie den Kleber 4 gewährleistet, während das Ausführungsbeispiel der Figuren 1 und 2 diesbezüglich auch das Releasetape 13 und die zugehörige Kleberschicht 14 nutzt.

Ergänzend weist das in Figur 6 dargestellte Ausführungsbeispiel noch ein Verklebungs- oder Kaschierband 32 auf, was jedoch, wie anhand der Erläuterungen des vorstehenden Absatzes unmittelbar nachvollziehbar, nicht zwingend vorgesehen sein muss. Dieses Verklebungs- oder Kaschierband 32 ist im Übergangsbereich 31 an der Kleberschicht 14 des Releasetapes 13 angeordnet und überlappt das zum Befestigungsbereich 9 weisende Ende des Releasetapes 13. Bei der Applikation an der Windel kann dieses Verklebungs- oder Kaschierband 32 um den Endbereich 16 herum gelegt werden, wobei es bereits in der Rolle mit dem Kleber 4 in Kontakt kommt und auf diese Weise eine sichere Positionierung des Releasetapes 13 bei der Applikation gewährleistet.

Insoweit verbleibt auch das Verklebungs- oder Kaschierband 32 an der Windel bzw. um den Endbereich 16 angeordnet, wenn das Windelverschlussband 1 entsprechend der Darstellung nach Figur 1 gestreckt wird. Hierbei kann das Verklebungs- oder Kaschierband 32 - je nach konkreter Umsetzung aber auch je nach der Bandbreite innerhalb der vorgegebenen Toleranzen beidseits der Falthilfe 17 weiterhin mit dem Kleber 4 verbunden bleiben, so dass hierdurch der Übergangsbereich 31 definiert wird und das Verklebungs- oder Kaschierband 32 an der Integrität des Windelverschlussbands 1 in gestrecktem Zustand über den Kleber 4 unterstützend wirksam wird. Andererseits ist auch möglich, dass in diesem Fall keine ausreichende Klebeverbindung über den Kleber 4 gewährleistet ist, so dass diese Unterstützung nicht zur Geltung kommt.

**Bezugszeichenliste:**

| | | | |
|---|---|---|---|
| 1 | Windelverschlussband | 17 | Falthilfe |
| 2 | Trägerbahn | 18 | Materialschwächung |
| 3 | NonWoven | 19 | Materialtrennung |
| 4 | Kleber | 20 | Längserstreckung |
| 5 | mechanische Befestigungseinrichtung | 21 | Schnitte |
| 6 | Hakenmaterial | 22 | Stege |
| 7 | Fingerlift | 23 | Stärke |
| 8 | Nutzerbereich | 24 | Maschinenrichtung |
| 9 | Befestigungsbereich | 25 | Bandmaterial |
| 10 | Windelohr | 26 | Klebebereich |
| 11 | Außenseite | 27 | Querstreifen |
| 12 | Innenseite | 28 | Schnittlinien |
| 13 | Releasetape | 29 | Rolle |
| 14 | Kleberschicht | 30 | Wicklung |
| 15 | Y-Bond | 31 | Übergangsbereich |
| 16 | Endbereich | 32 | Verklebungs- oder Kaschierband |

## Patentansprüche

1. Windelverschlussband (1), ggf. mit einem NonWoven (3) als einer Lage (2), mit einem Befestigungsbereich (9) und mit einem Nutzerbereich (8), zu dem ggf. ein zwischen dem Befestigungsbereich (9) und dem Nutzerbereich (8) vorgesehener Zwischenbereich zugehörig ist, sowie mit einem Übergangsbereich (31) zwischen dem Befestigungsbereich (9) und dem Nutzerbereich (8), bei welchem der Befestigungsbereich (9) permanent an einer Windel anbringbar ist, indem er wenigstens teilweise an einer Stelle um einen Windelrand gefaltet werden kann, **dadurch gekennzeichnet, dass** der Übergangsbereich (31) eine Falthilfe (17) in Form einer Materialtrennung (19) oder, wenn eine Lage (2) ein NonWoven (3) ist, eine Materialschwächung (18), aufweist, wobei die Falthilfe (17) befestigungsbereichseitig weniger als 2 mm von einem Klebebereich (26) eines Releasetapes (13) entfernt angeordnet ist.

2. Windelverschlussband (1) nach Anspruch 1, **dadurch gekennzeichnet, dass** die Materialtrennung (19) das Band (1) in seiner Stärke (23) durchtrennt.

3. Windelverschlussband (1) nach einem der vorstehenden Ansprüche, **dadurch gekennzeichnet, dass** die Materialtrennung (19) in Maschinenrichtung (24) durch Stege (22) unterbrochen ist, wobei vorzugsweise die Länge der Stege (22) in Maschinenrichtung (24) wenigstens 0,5% der Länge der Materialtrennung (19) beträgt.

4. Windelverschlussband (1) nach einem der Ansprüche 1 bis 3, **dadurch gekennzeichnet, dass** die Materialtrennung (19) ein Schnitt (21) ist.

5. Windelverschlussband (1) nach einem der vorstehenden Ansprüche, **dadurch gekennzeichnet, dass** die Falthilfe (17) befestigungsbereichseitig mehr als 0,3 mm, vorzugsweise mehr als 0,5 mm von einem Klebebereich (26) eines Releasetapes (13) entfernt angeordnet ist.

6. Windelverschlussband (1) nach einem der vorstehenden Ansprüche, **dadurch gekennzeichnet, dass** die Falthilfe (17) befestigungsbereichseitig weniger als 1 mm von einem Klebebereich (26) eines Releasetapes (13) entfernt angeordnet ist.

7. Windelverschlussband (1) nach einem der vorstehenden Ansprüche, **dadurch gekennzeichnet, dass** der Übergangsbereich (31) zumindest im Bereich der Falthilfe (17) permanent an der Windel befestigt ist.

8. Windel mit einem Windelverschlussband (1) nach einem der vorhergehenden Ansprüche.

9. Windel nach Anspruch 8, **dadurch gekennzeichnet, dass** die Falthilfe (15) an einem Windelrand der Windel anliegt.

10. Bandmaterial (25) zur Herstellung eines Windelverschlussbandes (1) nach einem der Ansprüche 1 bis 7, indem Querstreifen (27) von dem Bandmaterial (25) abgeschnitten werden können, **dadurch gekennzeichnet, dass** das Bandmaterial (25) eine Falthilfe (17) in Form einer längs (24) des Bandes (25) laufenden Materialtrennung (19) aufweist.

11. Bandmaterial (25) zur Herstellung eines Windelverschlussbandes (1) nach einem der Ansprüche 1 bis 7, indem Querstreifen (27) von dem Bandmaterial (25) abgeschnitten werden können, **dadurch gekennzeichnet, dass** das Bandmaterial (25) eine Falthilfe (17) in Form einer längs des Bandmaterials (25) laufenden Materialschwächung (18) von mindestens 50% aufweist.

12. Bandmaterial (25) mit einem NonWoven (3) als eine Lage zur Herstellung eines Windelverschlussbandes (1) nach einem der Ansprüche 1 bis 7, indem Querstreifen (27) von dem Bandmaterial (25) abgeschnitten werden können, insbesondere auch nach Anspruch 11, **gekennzeichnet durch** eine Falthilfe (17) in Form einer längs des Bandmaterials (25) laufenden Materialschwächung (18).

13. Bandmaterial (25) nach Anspruch 11 oder 12, **dadurch gekennzeichnet, dass** die Materialschwächung (18) eine Materialtrennung (19) ist.

14. Bandmaterial (25) nach Anspruch 10 oder 13, **dadurch gekennzeichnet, dass** die Materialtrennung (19) ein Schnitt (21) ist.

15. Wicklung (30) eines Bandmaterials (25) nach einem der Ansprüche 10 bis 14, wobei das Bandmaterial (25) in Maschinenrichtung (24) gewickelt ist.

## Claims

1. Nappy closing strip (1), optionally comprising a nonwoven (3) as one layer (2), comprising a fastening region (9) and comprising a user region (8), to which optionally an intermediate region provided between the fastening region (9) and the user region (8) belongs, and comprising a transition region (31) between the fastening region (9) and the user region (8) in which the fastening region (9) can be permanently attached to a nappy whereby said region can be folded about a nappy edge at least partially at one location, **characterised in that** the transition region (31) comprises a folding assistance (17) in the form of a material separation (19) or, if a layer (2) is a nonwoven (3), a material weakening (18), wherein the folding assistance (17) on the fastening region side is located at a distance of less than 2 mm from an adhesive region (26) of a release tape (13).

2. The nappy closing strip (1) according to claim 1, **characterised in that** the material separation (19) separates the strip (1) in its thickness (23).

3. The nappy closing strip (1) according to any one of the preceding claims, **characterised in that** the material separation (19) is interrupted in the machine direction (24) by webs (22), wherein preferably the length of the webs (22) in the machine direction (24) is at least 0.5% of the length of the material separation (19).

4. The nappy closing strip (1) according to any one of claims 1 to 3, **characterised in that** the material separation (19) is a cut (21).

5. The nappy closing strip (1) according to any one of the preceding claims, **characterised in that** the folding assistance (17) on the fastening region side is located at a distance of more than 0.3 mm, preferably more than 0.5 mm from an adhesive region (26) of a release tape (13).

6. The nappy closing strip (1) according to any one of the preceding claims, **characterised in that** the folding assistance (17) on the fastening region side is located at a distance of less than 1 mm from an adhesive region (26) of a release tape (13).

7. The nappy closing strip (1) according to any one of the preceding claims, **characterised in that** the transition region (31) is fastened permanently to the nappy at least in the region of the folding assistance (17).

8. Nappy having a nappy closing strip (1) according to any one of the preceding claims.

9. The nappy according to claim 8, **characterized in that** the folding assistance (15) abuts against a nappy edge of the nappy.

10. Strip material (25) for producing a nappy closing strip (1) according to one of claims 1 to 7, whereby transverse strips (27) can be cut from the strip material (25), **characterised in that** the strip material (25) has a folding assistance (17) in the form of a material separation (19) running along (24) the strip (25).

11. Strip material (25) for producing a nappy closing strip (1) according to one of claims 1 to 7, whereby transverse strips (27) can be cut from the strip material (25), **characterised in that** the strip material (25) has a folding assistance (17) in the form of a material weakening (18) of at least 50% running along the strip material (25).

12. Strip material (25) comprising a nonwoven (3) as one layer for producing a nappy closing strip (1) according to one of claims 1 to 7, whereby transverse strips (27) can be cut from the strip material (25), in particular according to claim 11, **characterised by** a folding assistance (17) in the form of a material weakening (18) running along the strip material (25).

13. The strip material (25) according to claim 11 or 12, **characterised in that** the material weakening (18) is a material separation (19).

14. The strip material (25) according to claim 10 or 13, **characterised in that** the material separation (19) is a cut (21).

15. Coil (30) of a strip material (25) according to any one of claims 10 to 14, wherein the strip material (25) is wound in the machine direction (24).

## Revendications

1. Bande de fermeture de couche (1), comportant le cas échéant un non-tissé (3) sous forme d'une épaisseur (2), avec une zone de fixation (9) et une zone utilisateur (8), dont fait partie le cas échéant une zone intermédiaire prévue entre la zone de fixation (9) et la zone utilisateur (8), ainsi qu'une zone de transition (31) entre la zone de fixation (9) et la zone utilisateur (8) et dans laquelle la zone de fixation (9) peut être appliquée de manière permanente sur une couche par le fait qu'elle peut être pliée au moins partiellement à un endroit autour d'un bord de la couche, **caractérisée en ce que** la zone de transition (31) présente une aide au pliage (17) sous forme d'une division de matériau (19) ou, si une épaisseur (2) est un non-tissé (3), un affinement du matériau (18), l'aide au pliage (17) étant disposée, au niveau de la zone de fixation, à moins de 2 mm d'une zone de collage (26) d'une bande de détachement (13).

2. Bande de fermeture de couche (1) selon la revendication 1, **caractérisée en ce que** la division de matériau (19) divise la bande (1) dans son épaisseur (23).

3. Bande de fermeture de couche (1) selon une des revendications précédentes, **caractérisée en ce que** la division de matériau (19) est interrompue dans le sens de la machine (24) par des traverses (22), la longueur des traverses (22) dans le sens de la machine (24) représentant de préférence au moins 0,5 % de la longueur de la division de matériau (19).

4. Bande de fermeture de couche (1) selon une des revendications 1 à 3, **caractérisée en ce que** la division de matériau (19) est une incision (21).

5. Bande de fermeture de couche (1) selon une des revendications précédentes, **caractérisée en ce que** l'aide au pliage (17) est disposée, au niveau de la zone de fixation, à plus de 0,3 mm, de préférence plus de 0,5 mm, d'une zone de collage (26) d'une bande de détachement (13).

6. Bande de fermeture de couche (1) selon une des revendications précédentes, **caractérisée en ce que** l'aide au pliage (17) est disposée, au niveau de la zone de fixation, à moins de 1 mm d'une zone de collage (26) d'une bande de détachement (13).

7. Bande de fermeture de couche (1) selon une des revendications précédentes, **caractérisée en ce que** la zone de transition (31) est fixée de manière permanente à la couche au moins au niveau de l'aide au pliage (17).

8. Couche comportant une bande de fermeture de couche (1) selon une des revendications précédentes.

9. Couche selon la revendication 8, **caractérisée en ce que** l'aide au pliage (15) est posée sur un bord de la couche.

10. Matériau de bande (25) pour la fabrication d'une bande de fermeture de couche (1) selon une des revendications 1 à 7, dans lequel des bandes transversales (27) de matériau de bande (25) peuvent être découpées, **caractérisé en ce que** le matériau de bande (25) présente une aide au pliage (17) sous forme d'une division de matériau (19) s'étendant le long (24) de la bande (25).

11. Matériau de bande (25) pour la fabrication d'une bande de fermeture de couche (1) selon une des revendications 1 à 7, dans lequel des bandes transversales (27) de matériau de bande (25) peuvent être découpées, **caractérisé en ce que** le matériau de bande (25) présente une aide au pliage (17) sous forme d'un affinement de matériau (18) d'environ 50 % suivant la longueur du matériau de bande (25).

12. Matériau de bande (25) comportant un non-tissé (3) sous forme d'épaisseur pour la fabrication d'une bande de fermeture de couche (1) selon une des revendications 1 à 7, dans lequel des bandes transversales (27) de matériau de bande (25) peuvent être découpées, en particulier aussi selon la revendication 11, **caractérisé par** une aide au pliage (17) sous forme d'un affinement de matériau (18) suivant la longueur du matériau de bande (25).

13. Matériau de bande (25) selon la revendication 11 ou 12, **caractérisé en ce que** l'affinement de matériau (18) est une division de matériau (19).

14. Matériau de bande (25) selon la revendication 10 ou 13, **caractérisé en ce que** la division de matériau (19) est une incision (21).

15. Enroulement (30) d'un matériau de bande (25) selon la revendication 10 à 14, le matériau de bande (25) étant enroulé dans le sens de la machine (24).
